# EUROPÄISCHE PATENTSCHRIFT

(11) **EP 3 665 091 B1**
(45) Veröffentlichungstag und Bekanntmachung des Hinweises auf die Patenterteilung: **09.08.2023**
(21) Anmeldenummer: 18734518.6
(22) Anmeldetag: 25.06.2018
(51) Int. Cl.: B65D 5/74, B65D 51/24, B65D 79/02, G01N 33/14

(54) **PACKUNG MIT EINEM WIEDERVERSCHLIESSBAREN ÖFFNUNGSELEMENT UND EINEM INDIKATORELEMENT SOWIE VERFAHREN ZUR HERSTELLUNG EINER PACKUNG**
PACKAGE COMPRISING A RECLOSABLE OPENING ELEMENT AND AN INDICATOR ELEMENT AND METHOD FOR PRODUCING A PACKAGE
EMBALLAGE COMPRENANT UN ÉLÉMENT D'OUVERTURE REFERMABLE ET UN ÉLÉMENT INDICATEUR ET PROCÉDÉ DE FABRICATION D'UN EMBALLAGE

(30) Priorität: 07.08.2017 DE 102017117891
(43) Veröffentlichungstag der Anmeldung: 17.06.2020
(73) Patentinhaber: PolyTaksys GmbH, 54340 Longuich (DE)
(72) Erfinder: REISERT, Steffen, 52066 Aachen (DE); HAUSER, Philippe, 8200 Schaffhausen (CH); MALINDRETOS, Lars, 47804 Krefeld (DE); RENNERS, Philip, 48147 Münster (DE)
(74) Vertreter: Fabry, Bernd
(86) Internationale Anmeldenummer: PCT/EP2018/066853
(87) Internationale Veröffentlichungsnummer: WO 2019/029892

(56) Entgegenhaltungen:
- DE-A1-102004 019 427
- DE-U1-202009 011 274
- GB-A- 2 344 101
- US-A1- 2006 256 664
- US-A1- 2017 087 524

## Beschreibung

### GEBIET DER ERFINDUNG

Die Erfindung betrifft eine Packung, insbesondere flüssigkeitsdichte Karton/Kunststoff-Verbundpackung für gießfähige Lebensmittelprodukte, mit einem wiederverschließbaren Öffnungselement, wobei das Öffnungselement auf die Packung appliziert ist und/oder einen integralen Bestandteil der Packung bildet, sowie Verfahren zur Herstellung einer solchen Packung.

### TECHNOLOGISCHER HINTERGRUND

Packungen für gießfähige Lebensmittelprodukte sind seit langem in vielfältigen Ausgestaltungen aus der Praxis bekannt. Hierzu werden häufig Karton/Kunststoff-Verbundpackungen eingesetzt, welche eine Kartonschicht aufweisen, die der Packung die ausreichende Stabilität und Struktur verleiht, und bei der die innere und äußere Oberfläche der Kartonschicht mit dünnen Kunststoffschichten beschichtet sind. Die Herstellung geschieht in der Regel durch Laminieren. Das Packungslaminat kann dabei weitere Schichten und Packstoffe aufweisen. Beispielsweise enthält es eine Aluminiumschicht als Barriereschicht gegen Licht und Sauerstoff.

Packungslaminate werden als Endlosbahn hergestellt, die als Rollenware zu Rollen aufgewickelt wird. Entweder werden die Packungen aus Zuschnitten hergestellt, die mittels einer Längsnaht zunächst zu einem Packungsmantel werden, welcher, flach gefaltet, besonders platzsparend ist, falls die "Packungsrohlinge" nach ihrer Herstellung erst zu einem Abfüllbetrieb transportiert werden müssen. Flach gefaltete Packungsmäntel lassen sich nahezu ohne Leervolumen in großer Anzahl auf Paletten transportieren. Alternativ werden Packungen jedoch auch aus Rollenware hergestellt, wobei schmalere Laminatrollen, deren Breite im Wesentlichen dem Packungsumfang entspricht in einer sogenannten "Schlauchformungsmaschine" zunächst zu einem Schlauch geformt werden, wobei der Schlauch dann unmittelbar nach seiner Herstellung mit Produkt befüllt wird und erst dann einzelne Packungen durch Siegeln der Quernähte kissenförmig hergestellt werden, welche in einem letzten Schritt in ihre spätere Packungsform gefaltet werden.

Unabhängig von den vorgenannten Herstellungsmethoden betrifft die vorliegende Erfindung sämtliche Lebensmittelpackungen, die mit einem Produkt mit begrenzter Haltbarkeit gefüllt sind.

Zur Information des Endverbrauchers werden Packungen neben dem grundsätzlichen Druckbild enthaltend die Beschreibung des Produktes, den Abfüllbetrieb, Abfüllort und dergleichen auch noch individuelle Daten aufgedruckt werden, wobei hier meist das Abfülldatum und in jedem Fall das sog. 'Mindesthaltbarkeitsdatum' (MHD) auf die Packung aufgedruckt werden. Damit weiß der Verbraucher, wie lange der Packungsinhalt, also das ihn interessierende Produkt - ungeöffnet - aufbewahrt werden kann.

Da die Lebensmittelpackungen in der Regel jedoch vor dem Ablauf des Mindesthaltbarkeitsdatums geöffnet werden, bieten konventionelle Packungen dem Verbraucher nach dem erstmaligen Öffnen keine Hilfestellung mit Bezug auf die Haltbarkeit des in der Packung befindlichen Produkts. Auch wenn es sich bei den in Rede stehenden Packungen um relativ kleine Gebinde handelt, sollte der Packungsinhalt - je nach Produkt - innerhalb weniger Tage bzw. Wochen aufgebraucht werden.

### RELEVANTER STAND DER TECHNIK

DE 10 2004 019427 A1 (KARDA) offenbart eine Kartonverpackung für flüssige Lebensmittel, die nach dem Öffnen dem Verbraucher eine Information über den Zustand des Produktes vermittelt. Konkret wird beim Ausgießen ein Messpunkt benetzt, der den pH-Wert bestimmt und diesen mit Hilfe einer Farbreaktion wiedergibt. Entsprechend dem jeweiligen Farbton und einer auf der Packung angebrachten Skalierung kann der Verbraucher dann erkennen, ob das Lebensmittel noch genießbar ist oder eher nicht.

GB 2344101 A (TAYLOR) betrifft einen Verschluss für einen Behälter umfassend eine Zeitgebereinrichtung und eine Einrichtung zum Erzeugen eines akustischen oder sichtbaren Warnsignals, wenn die Zeitgebereinrichtung anzeigt, dass seit dem Auftreten eines Auslöseereignisses ein vorbestimmtes Zeitintervall verstrichen ist. Das Auslöseereignis kann die erste Versiegelung des Behälters durch einen Hersteller sein, um ein "Mindesthaltbarkeitsdatum" zu signalisieren, oder das erste Öffnen des Behälters durch einen Benutzer, um ein "Gebrauch nach dem Öffnen"-Datum zu signalisieren.

US 2006 256664 A1(VARON) beansprucht eine Warnvorrichtung zum Anbringen an einer Verpackung eines Produkts, das ein Verfallsdatum hat, wobei die Vorrichtung eine Zeitgebereinrichtung zum Zählen des Ablaufs einer voreingestellten Zeitspanne gemäß einem auf das Produkt zugeschnittenen Zeitgeberprogramm bis zum Verfallsdatum und zum Bereitstellen eines Signals am Verfallsdatum und einen Indikator, der so angeordnet ist, dass er das Signal empfängt und eine Anzeige zum Unterscheiden einer Zeitspanne vor dem Verfallsdatum von einer Zeitspanne nach dem Verfallsdatum bereitstellt, und einen Schalter, der mit der Verpackung gekoppelt und so angeordnet ist, das er den Indikator aktiviert, wenn eine Person versucht, das Produkt zu benutzen, umfasst.

Für sich ist es aus der WO 2012/017244 A1 (OAKLEY) bereits bekannt, das Öffnungselement einer Packung der in Rede stehenden Art derart auszubilden, dass dieses eine verstellbare Anzeige zur Darstellung der Tage seit der Erstöffnung enthält. Eine solche Anzeige bedarf jedoch der Mithilfe des Verbrauchers. Wird die Anzeige vom Verbraucher nicht oder falsch eingestellt, ist auch dieser bereits bekannte Anzeigemodus ohne jeglichen Wert. Des Weiteren lassen sich Manipulationen (bewusst oder unbewusst) nicht zuverlässig verhindern.

### AUFGABE DER ERFINDUNG

Der vorliegenden Erfindung liegt daher die Aufgabe zu Grunde, die bekannten und auf dem Markt befindlichen Packungen mit gießfähigen Lebensmittelprodukten so auszugestalten und weiterzubilden, dass dem Verbraucher nach dem erstmaligen Öffnen der Packung der Zustand des Produktes signalisiert wird.

### BESCHREIBUNG DER ERFINDUNG

Ein erster Gegenstand der Erfindung betrifft eine Packung, insbesondere flüssigkeitsdichte Karton/Kunststoff-Verbundpackung für gießfähige Lebensmittelprodukte, mit einem wiederverschließbaren Öffnungselement, wobei das Öffnungselement außen auf die Packung appliziert ist und/oder einen integralen Bestandteil der Packung bildet, sowie
(a) im Bereich des Öffnungselements ein aktives Indikatorelement vorgesehen ist, welches dem Verbraucher Informationen über den Zustand und/oder die Haltbarkeit des in der Packung befindlichen Produktes anzeigt,
(b) das Indikatorelement ein Aktivierungselement aufweist, welches derart in oder am Öffnungselement angeordnet ist, dass das Indikatorelement beim erstmaligen Öffnen der Packung durch Betätigung des Öffnungselements aktiviert wird, welche sich dadurch auszeichnet, dass
(c) das Aktivierungselement gleichzeitig als Messelement dient,
(d) das Aktivierungselement durch mechanische Bewegung zur Herbeiführung eines elektrischen Kontakts ausgelöst wird, der einen elektrochemischen Prozess in Gang setzt, und
(e) das Indikatorelement wenigstens mittelbar durch einen oder mehrere der Parameter Zeit, Temperatur, pH-Wert, Sauerstoffgehalt und/oder durch Bio- oder Chemie-Rezeptoren beeinflussbar ist.

Unter einem aktiven Indikatorelement werden solche Indikatorelemente verstanden, welche nach einer ersten Aktivierung, vorzugsweise durch das erstmalige Öffnen der damit ausgestatteten Packung, dem Verbraucher Informationen über den Zustand und/oder die Haltbarkeit des in der Packung befindlichen Produktes anzeigen. Man könnte also auch von "aktivierbaren" Indikatorelementen sprechen.

Gemäß einem Ausführungsbeispiel wird vorgeschlagen, dass das aktive Indikatorelement eine chemische Zelle aufweist, und dass beim erstmaligen Öffnen der Packung die chemische Zelle aktiviert wird. Eine chemische Zelle kann beispielsweise auf einer Redoxreaktion basieren. Hierbei kann ein Elektrolyt mit einem Reaktionspartner reagieren. Beispielsweise kann als Elektrolyt NaCl dienen und als Reaktionspartner ein nanoskaliges Aluminium. Wird die Reaktion angestoßen, verläuft diese nach dem Prinzip der lateralen Oxidation bis zum Verbrauch des Reaktionspartners ab. Beispielsweise kann sie als eine laterale Oxidation einer Aluminiumschicht ablaufen. Diese und andere chemische Reaktionen können entlang einer Schicht des Reaktionspartners verlaufen. Hierbei kann beispielsweise eine dünne Schicht eines Reaktionspartners mit einem Elektrolyt dem elektrochemischen Prozess unterworfen werden. Durch diesen Prozess kann eine unter der Schicht des Reaktionspartners liegende Information optisch freigegeben werden. Eine solche Information kann graphische Symbole, Buchstaben und/oder aber auch Zahlen enthalten, die dem Verbraucher klare und eindeutige Informationen über den Zustand und/oder die Haltbarkeit des in der Packung befindlichen Produktes anzeigt.

Eine weitere Lehre der Erfindung sieht vor, dass das aktive Indikatorelement ein Aktivierungselement aufweist, welches derart in oder am Öffnungselement angeordnet ist, dass das Indikatorelement beim erstmaligen Öffnen der Packung durch Betätigung des Öffnungselements aktiviert wird. Erfindungsgemäß erfolgt die Aktivierung durch eine mechanische Bewegung, beispielsweise des Öffnungselementes oder eines Pulltabs, zur Herbeiführung eines elektrischen Kontakts. Beispielsweise kann dies mittels Kurzschließen zweier Kontakte beim Aufdrehen der Schraubkappe eines Öffnungselements mittels eines entsprechenden in der Schraubkappe vorgesehenen Leiters oder mittels Betätigen des Pulltabs geschehen.

Gemäß einem Ausführungsbeispiel ist die Reaktion selbstversorgend, so dass diese nachdem sie aktiviert wurde, bis zum Verbrauch des Reaktionspartners abläuft. Hierbei kann beispielsweise von dem Ursprung der Reaktion entlang der Schicht des Reaktionspartners die Reaktion in einer Raumrichtung mit einer scharf abgegrenzten Front verlaufen. Das unter dem Reaktionspartner, beispielsweise der Aluminiumschicht, liegende Substrat wird dabei beispielsweise optisch freigelegt, so dass eine dort aufgedruckte Information nach und nach erkennbar wird. Mit dieser Information kann beispielsweise eine Information zu der Haltbarkeit des Produkts angegeben werden. Wird durch die Aktivierung der chemische Prozess in Gang gesetzt, so löst sich nach und nach der Reaktionspartner, beispielsweise die Aluminiumschicht, auf und nach und nach wird das darunterliegende Label freigelegt. Auf diesem Label kann die Information zu der Haltbarkeit angegeben werden und bei einem vollständigen Verbrauch kann beispielsweise dem Verbraucher eine Information angezeigt sein, dass das Lebensmittel nicht mehr haltbar ist.

Gerade bei elektrochemischen Prozessen kann eine erstmalige Aktivierung das Indikatorelement dauerhaft aktivieren. Dauerhaft bedeutet in diesem Fall, dass die Aktivierung länger ist, als die eigentliche Initialaktivierung durch das Kurzschließen dauert. In dem oben genannten Beispiel kann beispielsweise die Dauerhaftigkeit durch die selbstablaufende Reaktion zwischen dem Elektrolyten und dem Reaktionspartner gegeben sein. Ist der Reaktionspartner aufgebraucht, so ist die Aktivierung beendet, was jedoch nach wie vor unter den Begriff dauerhafte Aktivierung fällt.

Die Anzeige auf den vorgenannten Indikatorelementen kann dabei auf unterschiedlichste Weise realisiert sein. Beispielsweise lassen sich bei einfachen Indikatoren Farben oder Farbverläufe anzeigen, welche sich über den Verbrauchszeitraum einstellen bzw. verändern. Etwas aufwendigere Indikatoren können auch über ein Display verfügen, auf dem die Anzahl der Tage seit Öffnung oder - umgekehrt die noch verbleibenden Haltbarkeitstage in entsprechender wechselnder Reihenfolge dargestellt werden können.

Eine weitere Lehre der Erfindung sieht vor, dass das Indikatorelement und das Aktivierungselement einstückig ausgebildet sind. Diese Lösung ist besonders zweckmäßig, da sich Indikatorelement und Aktivierungselement stets im richtigen Abstand und in richtiger Ausrichtung zueinander und mittels entsprechender verbauter Leiterbahnen als Ganzes in einem einzigen Arbeitsgang mit der Packung oder dem Öffnungselement verbinden lassen, so dass bei der Packungsherstellung die für konventionelle Packungen vorhandenen Maschinen etc. weiter eingesetzt werden können.

Erfindungsgemäß weist das Indikatorelement ein Aktivierungselement auf, das gleichzeitig als Messelement dient, welches weitere Parameter wie Temperatur, Feuchtigkeit, od. dgl. misst und über das Indikatorelement anzeigt. Besonders bevorzugt sind das Indikatorelement und das Messelement einstückig ausgebildet. Als Messelement kann beispielsweise wenigstens ein Sensor verwendet werden.

Bei dem Sensor kann es sich beispielsweise um einen Drucksensor, einen pH-Wert-Sensor, einen Temperatursensor, einen Sensor zur Messung des Sauerstoffgehaltes, einen Sensor zur Messung der elektrischen Leitfähigkeit, einen Sensor zur Messung des Anteils eines oder mehrerer Vitamine/Spurenelemente oder einen Sensor zur Messung von Stoffwechselprodukten handeln. Ein Sensor (auch: "Aufnehmer") dient dem Zweck, bestimmte physikalische oder chemische Eigenschaften des Inhalts der Verpackung qualitativ und/oder quantitativ zu erfassen. Es kann sich um einen passiven Sensor (benötigt keine Hilfsenergie) oder um einen aktiven Sensor (benötigt Hilfsenergie) handeln. Die Hilfsenergie kann durch eine integrierte Batterie bereitgestellt werden oder durch eine externe Aktivierung (z.B. durch elektromagnetische Induktion) bereitgestellt werden. Je nach der zu messenden Größe und dem anzuwendenden Messprinzip kann der Sensor an der Oberfläche, insbesondere an der Innenseite des Kunststoffelements angeordnet sein (beispielsweise für berührende Messverfahren) oder in das Kunststoffelement integriert sein (beispielsweise für berührungslose Messverfahren). Es ist von Vorteil, wenn zur Ermittlung der augenblicklich vorliegenden Qualität des Lebensmittels zwei Messwerte herangezogen werden. Dabei kann es sich um denselben Parameter handeln, wobei der Sensor den Messwert, beispielsweise einen pH-Wert, erstmalig , beispielsweise beim Inkontakttreten mit dem Produkt, misst und danach eine Veränderung des Messwertes, ohne den tatsächlichen quantitativen Wert zu kennen.

Der Sensor ist daher bevorzugt ein Sensor, der eine qualitative Veränderung eines Messwertes erfasst. Daher kann es fallweise von Vorteil sein, wenn der Sensor zur Ermittlung wenigstens eines Relativwertes des Messwertes relativ zu einem initialen Messwert gebildet ist.

Es sind zudem Ausführungen von teilweise bzw. vollständig druckbaren Sensoren denkbar, welche auf dem Prinzip eines lonensensitive Feldeffekttransistors (ISFET) bzw. Electrolyte-Insulator-Semiconductor (EIS) beruhen. Hierbei werden die Standardmaterialien der Siliziumtechnologie ganz oder teilweise durch funktionale Tinten auf Basis von Polymeren ersetzt, welche die gleichen bzw. ähnliche elektrische bzw. elektrochemische Eigenschaften aufweisen wie die herkömmlichen Materialien. Die Strukturen werden dabei mit unterschiedlichen Druckverfahren, z.B. Siebdruck oder Inkjet-Druck auf einem Substrat, insbesondere der Deckschicht erzeugt. Das Substrat kann dabei neben seiner Funktion als Träger der Sensorstruktur selbst als funktionaler Teil der Sensorstruktur dienen, beispielsweise als Isolator, Halbleiter oder ionensensitive Schicht. Als Substrat können dabei einschichtige, mehrschichtige, steife oder flexible Träger in Form von z.B. Folien, Platten (Plättchen), Bögen, Streifen od. dgl. dienen. Insbesondere die Kombination aus teilweise bzw. vollständig gedruckten Sensorstrukturen aus funktionalen Tinten auf flexiblen Foliensubstraten als Träger ermöglicht eine kostengünstige Fertigung, sogar bei kleineren Stückzahlen.

Eine weitere Lehre der Erfindung sieht vor, dass das Indikatorelement wenigstens mittelbar durch einen oder mehrere der Parameter Zeit, Temperatur, pH-Wert, Sauerstoffgehalt od. dgl. und/oder durch Bio- oder Chemie-Rezeptoren beeinflussbar ist. Eine Information für den Verbraucher kann auch durch einen Hinweis gebildet sein, der auf wenigstens einem Zustand, insbesondere einem Zustandswert, oder einer Zustandsänderung, insbesondere einem Zustandsänderungswert eines Parameters und/oder eines Rezeptors der Einfluss auf die Eigenschaften des verpackten Produktes, insbesondere auf die Genießbarkeit des verpackten Produktes ausübt, basiert. Die Information kann in einem solchen Fall einen mittelbaren Hinweis auf den Zustand und/oder die Haltbarkeit des in der Packung befindlichen Produkts anzeigen.

Erfindungsgemäß kann das Indikatorelement zur Messung des pH-Werts, der Temperatur, des Sauerstoffgehalts, des Anteils eines oder mehrerer Vitamins/Spurenelements, der elektrischen Leitfähigkeit, von Stoffwechselprodukten oder dergleichen eingerichtet sein. Es kann von Vorteil sein, wenn das Indikatorelement zur Ermittlung wenigstens eines Absolutwertes gebildet ist.

Es ist von Vorteil, wenn zur Ermittlung der augenblicklich vorliegenden Qualität des Lebensmittelprodukts zwei Messwerte herangezogen werden. Dabei kann es sich um denselben Parameter handeln, wobei ein Sensor den Messwert, bspw. einen pH-Wert, erstmalig, beispielsweise beim Inkontakttreten mit dem Produkt, misst und danach eine Veränderung des Messwertes, ohne den tatsächlichen quantitativen Wert zu kennen. Der Sensor ist daher bevorzugt ein Sensor, der eine qualitative Veränderung eines Messwertes erfasst. Daher kann es fallweise von Vorteil sein, wenn der Sensor zur Ermittlung wenigstens eines Relativwertes des Messwertes relativ zu einem initialen Messwert gebildet ist.

Bevorzugt kann ein Sensor des Indikatorelements zur Messung des pH-Wertes dienen. Der pH-Wert ist ein Maß für den sauren oder basischen Charakter einer wässrigen Lösung. Er wird anhand der Wasserstoffionenaktivität der wässrigen Lösung ermittelt. Es gibt verschiedene Methoden den pH-Wert zu ermitteln. Eine Möglichkeit liegt in der Anwendung einer potentiometrischen Messkette (Potentiometrie). Hierbei wird das Potential, welches sich in direkter Abhängigkeit zur H⁺-Ionen-Konzentration an einer ionensensitiven Elektrode bildet, gemessen. Die Messung erfolgt dabei als Potentialdifferenzmessung gegenüber einer Bezugselektrode, auch Referenzelektrode genannt, welche ein konstantes Potential bereitstellt.

Mit Vorteil kann ein solcher Sensor auch als Temperatursensor ausgebildet sein. Dabei kann es sich fallweise mit Vorteil um einen aktiven Temperatursensor handeln. Aktive Temperatursensoren erzeugen aufgrund ihres Messprinzips ein elektrisches Signal. Dies hat den Vorteil, dass dabei keine elektrische Hilfsenergie benötigt wird. Ein Beispiel für einen solchen aktiven Temperatursensor ist das Thermoelement. Insbesondere in diesem Fall kann auch bereits ein elektrisch gegenüber der Barriereschicht isolierter Leiter ausreichend sein. Es ist jedoch auch möglich, dass der Temperatursensor als passiver Temperatursensor ausgebildet ist. Bei passiven Temperatursensoren wird im Gegensatz zu aktiven Sensoren eine Hilfsenergie benötigt, um das Signal auslesen zu können. Beispiel für einen passiven Temperatursensor ist ein Widerstandsthermometer.

Es wurde bereits darauf hingewiesen, dass das Indikatorelement so ausgebildet sein kann, dass es Informationen über die Zeit seit der Erstöffnung der Packung anzeigt. Dabei kann ein für viele Lebensmittel verfügbarer Erfahrungsschatz genutzt werden. So ist der Einfluss von Zeit in Abhängigkeit einer Temperaturkurve auf die Genießbarkeit von Milch eindeutig belegt. Ein bevorzugtes Ausführungsbeispiel kann in diesem Zusammenhang etwa ein Sichtfenster über einer darunter abgebildeten Farbskala über einen gewissen Zeitraum öffnen, wobei die Öffnungsgeschwindigkeit sich aktiv temperaturabhängig einstellt. Ein Farbbereich, der auf die Erreichung einer, wenigstens mit hoher Wahrscheinlichkeit vorliegenden, Ungenießbarkeit des Produktes hinweist wird nach einem gewissen Zeitraum erreicht, wobei die Größe des Zeitraums abhängig davon ist, welchen Temperaturen die Packung in diesem Zeitraum ausgesetzt war. Auch können in die Beeinflussung des Zeitraumes weitere Parameter und/oder Rezeptoren einfließen.

Um die Haltbarkeit des Lebensmittels abhängig von den Umgebungsbedingungen anzeigen zu können, kann es sinnvoll sein, dass die chemische Reaktion temperaturabhängig ist. Insbesondere kann die Verbrauchsgeschwindigkeit des Reaktionspartners temperaturabhängig sein. Beispielsweise kann bei einer niedrigen Temperatur die Reaktion langsamer ablaufen als bei einer hohen Temperatur. Entsprechende Elektrolyte und Reaktionspartner sind bekannt, so dass eine geeignete Auswahl erfolgen kann.

Es ist von besonderem Vorteil, wenn auch das Indikatorelement und das Messelement einstückig ausgebildet sind. Dabei dient das Aktivierungselement gleichzeitig als Messelement, um die Baugröße des Indikatorelements zu reduzieren.

Eine weitere Lehre der Erfindung sieht vor, dass das Indikatorelement als flaches Etikett ausgebildet ist, welches bevorzugt streifenförmig ausgebildet sein kann, insbesondere dann, wenn es sich um ein Indikatorelement mit einer Anzeige in einer Längserstreckung handelt.

Vorteilhaft ist auch, wenn das Indikatorelement selbstklebend ausgebildet ist, wobei es zweckmäßig ist, dass das Indikatorelement möglichst flach ausgeführt ist.

Nach einer anderen Ausgestaltung der Erfindung ist vorgesehen, dass das Öffnungselement eine Kavität aufweist, die wenigstens einen Teil des Indikatorelements aufnimmt. Eine solche Kavität kann beispielsweise bevorzugt im Bereich des Flansches des Öffnungselements ausgebildet sein, wobei das Indikatorelement teilweise oder vollständig in der Kavität angeordnet sein kann.

Je nach gewünschter Ausführungsform kann die eigentliche Darstellung der Informationen außerhalb des Öffnungselements erfolgen, wenn beispielsweise ein streifenförmiges Indikatorelement verwendet wird, dessen Anzeigeteil sich in dem aus dem Öffnungselement herausragenden Abschnitt befindet.

Eine andere bevorzugte Lehre sieht vor, dass das Verbundmaterial als Laminat mit einer aus einem Fasermaterial, insbesondere aus Papier, Pappe oder Karton, gebildeten, strukturgebenden Trägerschicht und äußeren Deckschichten aus Kunststoff gebildet ist und dass im Verbundmaterial ein Indikatorelement benachbart zu der Außenseite oder der Innenseite einer äußeren Deckschicht angeordnet ist. Es ist also erfindungsgemäß auch möglich, das Indikatorelement in das Verbundmaterial zu integrieren.

Eine andere Möglichkeit der Aufbringung des Indikatorelements ist bei Packungen mit Öffnungselementen denkbar, welche eine Schraubkappe aufweisen. Hier kann das Indikatorelement auf oder an der Schraubkappe ausgebildet sein und auf diese Weise mit dem Inneren des Öffnungselements in Verbindung stehen. Bevorzugt ist ein solches Indikatorelement außen an der Schraubkappe angeordnet und im Inneren der Schraubkappe ein Aktivierungselement und Messelement vorhanden, wobei die Verbindung zwischen Indikatorelement und Aktivierungs- bzw. Messelement über Leiterbahnen erfolgt, welche bei der Herstellung der Schraubkappe im Spritzwerkzeug beim Spritzvorgang der Schraubkappe mit Kunststoff umschlossen werden.

Falls das Indikatorelement vollständig im Inneren der Kavität des Öffnungselements oder der Schraubkappe angeordnet ist, kann der Anzeigeteil des Indikatorelements von einem Sichtfenster überdeckt sein oder ist es auch möglich, das Indikatorelement während der Herstellung des Öffnungselements im Spritzwerkzeug unmittelbar zu einem Teil des Flansches des Öffnungselements zu machen. Dabei kann der Anzeigeteil des Indikatorelements an der Oberseite des Flansches freiliegend angeordnet sein oder aber er ist komplett mit Kunststoffmaterial umhüllt, wobei dann ein transparenter oder wenigstens ein opaker Kunststoff verwendet werden sollte, um die darauf befindlichen Informationen auch durch den Kunststoff ablesbar zu machen.

### HERSTELLVERFAHREN

Ein weiterer Gegenstand der Erfindung betrifft ein erstes Herstellungsverfahren für eine erfindungsgemäße Packung, wobei das Verfahren folgende Schritte vorsieht:
- Herstellen einer Verpackung aus einem Zuschnitt oder einer Materialbahn aus Verbundmaterial, Befüllen der Verpackung mit einem Produkt;
- Verschließen der gefüllten Verpackung zu einer Getränke- oder Lebensmittelpackung,
- Aufbringen des Indikatorelements auf den Giebelbereich der Packung und
- Aufbringen des Öffnungselements auf die Packung, wobei das Indikatorelement ganz oder teilweise vom Öffnungselement überdeckt ist.

Ein weiterer Gegenstand der Erfindung betrifft ein zweites Herstellungsverfahren für eine erfindungsgemäße Packung, wobei das Verfahren folgende Schritte vorsieht:
- Herstellen einer Verpackung aus einem Zuschnitt oder einer Materialbahn aus Verbundmaterial,
- Befüllen der Verpackung mit einem Produkt,
- Verschließen der gefüllten Verpackung zu einer Getränke- oder Lebensmittelpackung,
- Einbringen des Indikatorelements in das Öffnungselement und
- Aufbringen des Öffnungselements auf die Packung.

### BESCHREIBUNG DER ZEICHNUNGEN

Nachfolgend wird die Erfindung anhand einer lediglich bevorzugte Ausführungsbeispiele darstellenden Zeichnung näher erläutert. In der Zeichnung zeigen
- Fig. 1A: Eine nicht-erfindungsgemäße Packung in perspektivischer Ansicht,
- Fig. 1B: ein Öffnungselement ohne Schraubkappe der Packung von Fig. 1A,
- Fig. 1C: einen Vertikalschnitt entlang der Linie IC-IC in Fig. 1A,
- Fig. 2A: ein erfindungsgemäßes Ausführungsbeispiel einer erfindungsgemäßen Packung,
- Fig. 2B: ein Öffnungselement ohne Schraubkappe der erfindungsgemäßen Packung von Fig. 2A,
- Fig. 2C: einen Vertikalschnitt entlang der Linie IIC-IIC in Fig. 2A,
- Fig. 2D: eine Modifikation des Öffnungselementes in der Darstellung aus Fig. 2C,
- Fig. 2E: eine weitere Modifikation des Öffnungselementes in der Darstellung aus Fig. 2C,
- Fig. 3A: ein weiteres Ausführungsbeispiel einer erfindungsgemäßen Packung,
- Fig. 3B: eine Schraubkappe für das Öffnungselement der in Fig. 3A dargestellten Packung,
- Fig. 3C: einen Vertikalschnitt entlang der Linie IIIC-IIIC in Fig. 3A,
- Fig.4A: ein Indikatorelement der erfindungsgemäßen Packung im Vertikalschnitt entlang der Linie IVA-IVA in Fig. 4B,
- Fig. 4B: das Indikatorelement der erfindungsgemäßen Packung im Horizontalschnitt entlang der Linie IVB-IVB in Fig. 4A,
- Fig. 4C: das Indikatorelement aus Fig. 4B nach seiner Aktivierung und
- Fig. 5: ein Diagramm mit der qualitativen Auswirkung der Temperatur auf die Anzeige des Indikatorelements.

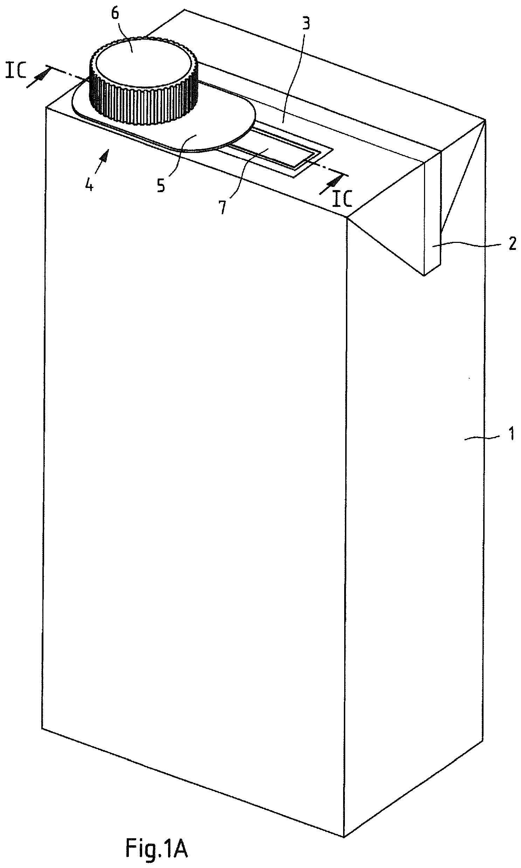

In Fig. 1A ist eine nicht-erfindungsgemäße Packung 1 dargestellt, welche im dargestellten Beispiel als quaderförmige Verbundpackung dargestellt ist. Diese Packung 1 weist an ihrer Oberseite eine umgelegte Giebelnaht 2 auf, die den Giebelbereich in zwei Teilflächen aufteilt, von denen die größere Fläche 3 ein Öffnunaselement 4 aufweist, welches mittels einem FJansch 5 mit der Packung 1 verbunden ist und eine Schraubkappe 6 aufweist. Im Bereich des Öffnungselements ist ein Indikatorelement 7 vorgesehen, welches dem Verbraucher Informationen über den Zustand und/oder die Haltbarkeit des in der Packung 1 befindlichen Produktes anzeigt. Der Aufbau und die Funktion des Indikatorelements 7 werden weiter unten näher beschrieben.

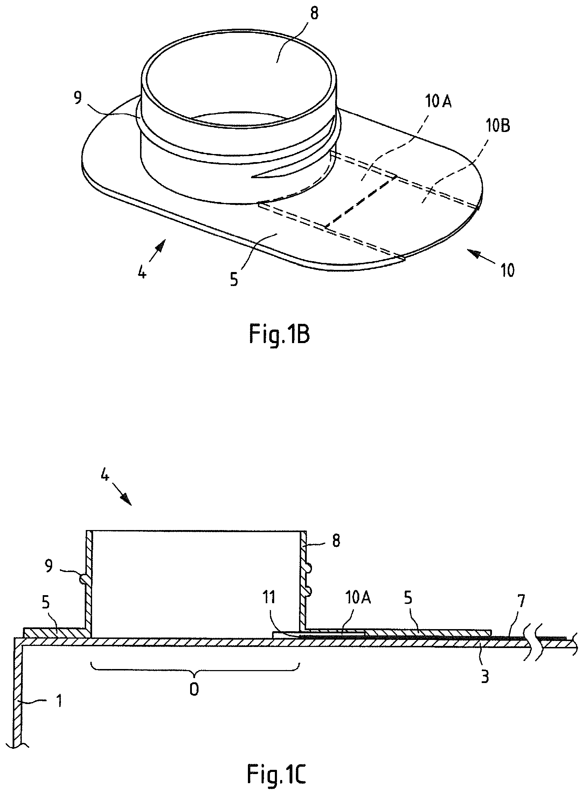

Fig. 1B zeigt in vergrößerter Darstellung das Öffnungselement 4 aus Fig. 1A. Zur besseren Übersicht ist das Öffnungselement 4 hier ohne Schraubkappe dargestellt. Man erkennt deutlich, dass sich vom Flansch 5 ausgehend ein zylinderförmiger Ausgießtubus 8 nach oben erstreckt, der auf seiner Außenseite ein Gewinde 9 zur Wirkverbindung mit der nicht dargestellten Schraubkappe aufweist. Ferner erkennt man, dass das Öffnungselement 4 eine Kavität 10 aufweist, welche im dargestellten und insoweit bevorzugten Ausführungsbeispiel gestuft ausgeführt ist und zwei Teilbereiche 10A und 10B mit unterschiedlicher Höhe aufweist.

In Fig. 1C ist das Öffnungselement 4 in seiner auf die Packung 1 applizierten Stellung im Vertikalschnitt entlang der Linie IC -IC aus Fig. 1A (ohne Schraubkappe) dargestellt. Hier zeigt sich deutlich die zweistufige Ausbildung der Kavität 10 im Öffnungselement 4. Man erkennt, dass der höhere Teilbereich 10A der Kavität 10 einen Luftspalt oberhalb des Indikatorelements 7 bildet, während der (aus Gründen der Übersicht nicht dargestellte) flachere Bereich 10B aus Fig. 1B) der Höhe des Indikatorelements 7 entspricht und dazu dient, dass dieses beim flächigen Aufbringen des Öffnungselements 4 auf die Packung 1 nicht aufträgt und zu Undichtigkeiten führen kann. Man erkennt darüber hinaus, dass das im Bereich der Kavität 10A befindliche Ende des Indikatorelementes 7 eine freie Oberfläche aufweist, welche als Aktivierungselement 11 wirkt. Dazu verfügt das Aktivierungselement 11 über einen feuchteabhängigen Sensor, wobei beim erstmaligen Öffnen die sich einstellende feuchte Atmosphäre ausreicht, um die Aktivierung des Indikatorelementes 7 zuverlässig vorzunehmen. Auch wenn im in Fig. 1C dargestellten Fall die Giebelfläche 3 der Packung 1 unterhalb des Öffnungselements intakt dargestellt ist, ist mit dem Bezugszeichen O der - spätere - Öffnungsbereich angedeutet, weil in diesem Bereich durch ein (nicht dargestelltes) Schneidelement beim erstmaligen Abschrauben der Schraubkappe eine Gießöffnung im Öffnungsbereich O erzeugt wird.

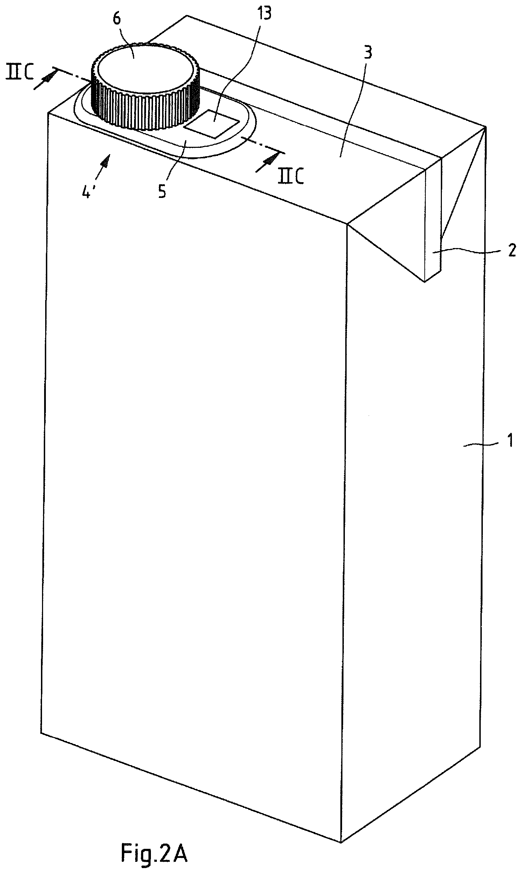

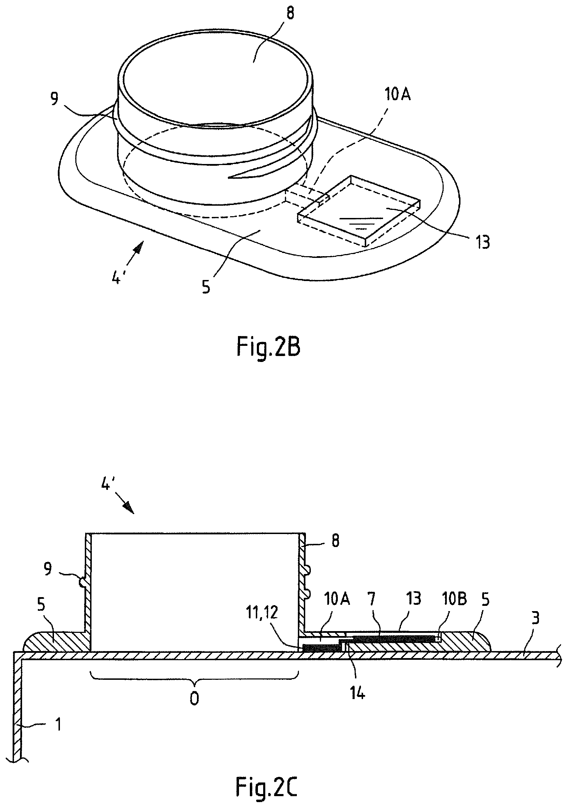

Die erfindungsgemäßen Fig. 2A, 2B und 2C entsprechen im Wesentlichen den Darstellungen in den Fig. 1A, 1B und 1C, wobei jedoch das Öffnungselement 4' modifiziert ist und das Aktivierungselement gleichzeitig das Messelement verkörpert. Hierbei ist das Indikatorelement 7 vollständig im Flansch 5 des Öffnungselements 4' enthalten, wie insbesondere aus Fig. 2C leicht erkennbar ist. Neben dem bereits zu Fig. 1C beschriebenen Aktivierungselement 11 enthält das freie Ende des Indikatorelements 7 auch ein Messelement 12, welches eine Messung weiterer Parameter wie Temperatur oder Feuchtigkeit über den Anzeigezeitraum durchführt und die Anzeige auf dem Indikatorelement 7 entsprechend beeinflusst. Zum Ablesen der Anzeige weist das Öffnungselement 4' in diesem Ausführungsbeispiel ein Sichtfenster 13 auf, welches unmittelbar oberhalb des Indikatorelementes 7 angeordnet ist und entweder eine in das Öffnungselement 4' flüssigkeitsdicht eingesetzte Sichtscheibe aufweist oder bei dem ein transparentes oder opakes Material für den Flansch 5 des Öffnungselements 4' verwendet wird. Das eigentliche Indikatorelement 7 ist dabei mittels Leiterbahnen 14 mit dem Aktivierungselement 11 und dem Messelement 12 verbunden. Das Sichtfenster 13 gehört dabei zur Kavität 10B.

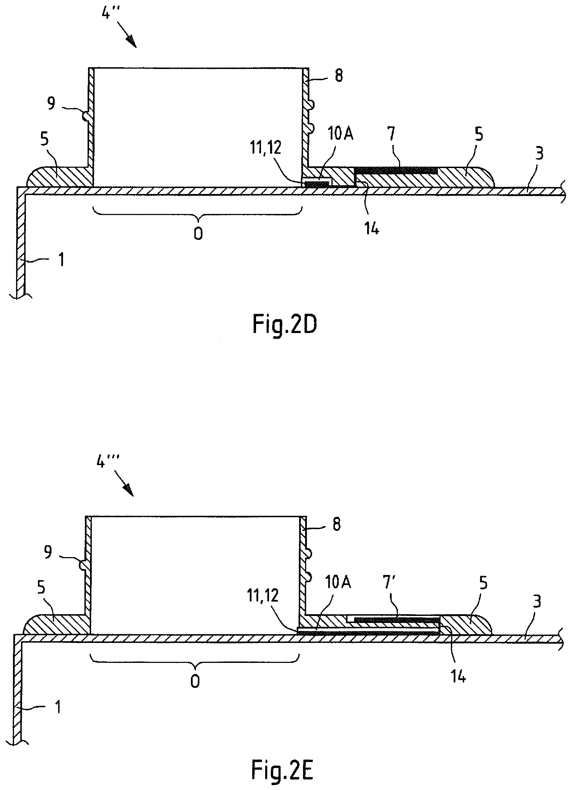

In Fig. 2D ist eine weitere Modifikation dargestellt, bei dem das Indikatorelement 7 beim Spritzvorgang des wiederum modifizierten Öffnungselements 4" im Spritzwerkzeug enthalten ist, sodass das Kunststoffmaterial die Leiterbahnen 14 umschließt und die Sichtfläche des Indikatorelementes 7 nach oben hin nicht bedeckt.

Fig. 2E zeigt schließlich eine weitere Modifikation, bei der ein Öffnungselement 4‴ verwendet wird, wobei eine relativ große Kavität 10A vorgesehen ist, und am Ende dieser Kavität Leiterbahnen 14 zwischen dem Aktivierungselement 11 sowie Messelement 12 und einem Indikatorelement 7' vorhanden sind, wobei das Indikatorelement 7' seine Anzeigeseite gewissermaßen auf der "Unterseite" des einstöckig hergestellten Indikatorelements 7 hat, welche dann im Spritzwerkzeug um 180° umgeklappt wird.

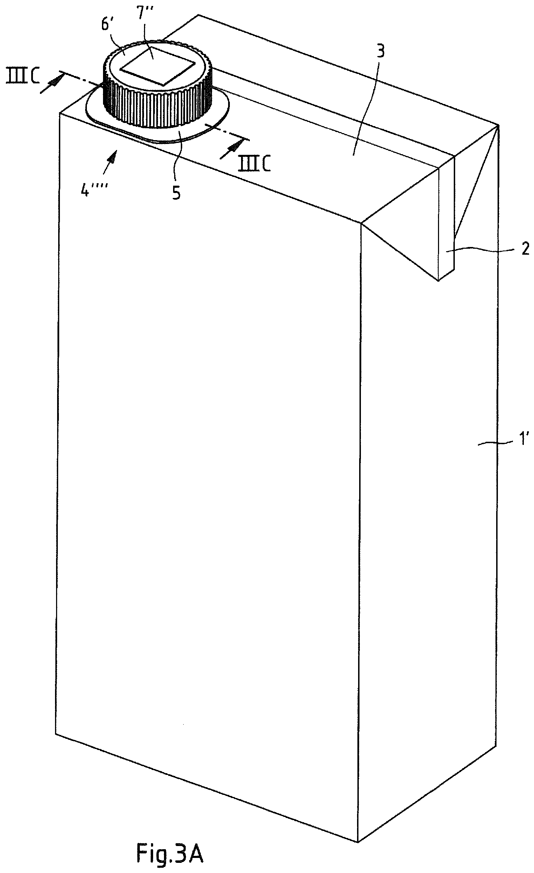

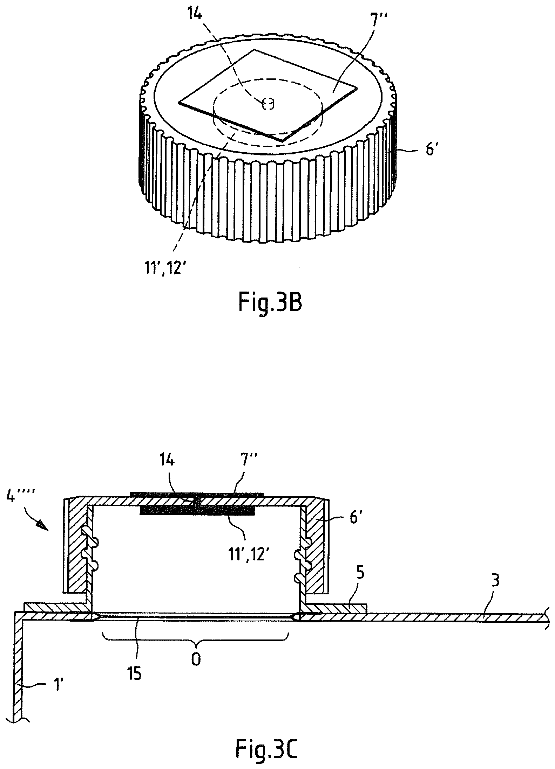

Auch die Fig. 3A-3C eines weiteren Ausführungsbeispiels entsprechen im Wesentlichen den Darstellungen der Fig. 1A-1C bzw. 2A-2C. Hierbei ist ein Indikatorelement 7" gezeigt, das nicht unmittelbar auf die Packung 1' appliziert wurde, sondern in der Schraubkappe 6' des Öffnungselements 4 angeordnet ist, wobei der Anzeigeteil des Indikatorelements 7" oben auf der Oberseite der Schraubkappe 6' aufgebracht ist und mittels Leiterbahnen 14 mit einem im Inneren der Schraubkappe 6' befindlichen Aktivierungselement 11' und Messelement 12' in Verbindung steht. Bei diesem Ausführungsbeispiel muss das Basisteil des Öffnungselementes 4ʺʺ gegenüber einem konventionellen Öffnungselement überhaupt nicht modifiziert werden.

In Fig. 3C ist ferner der Öffnungsbereich O der Packung 1' als sog. "überbeschichtetes Loch" 15 ausgeführt. Hierbei handelt es sich um eine runde Öffnung im Kartonmaterial der Packung 1', welche beim Laminieren mit einer (doppelten) Kunststoffschicht überbeschichtet wurde, wobei sich die beiden Kunststofffilme zu einer einzigen Schicht verbinden. Im Inneren des Öffnungselementes 4, 4' befindet sich ein (nicht dargestellter) Schneidring, welcher beim Abschrauben der Schraubkappe 6' durch entsprechende (nicht dargestellte) Mitnehmer angetrieben wird und über ein gegenläufiges Innengewinde im Ausgießtubus nach unten gedreht wird, wobei sich ein oder mehrere Schneidzähne in die Folie des überbeschichteten Lochs 15 drehen und dort mittels einer rotierenden Schneidbewegung die Erstöffnung der Packung 1' vornehmen. Aus Gründen der Übersichtlichkeit sind weder der Schneidring noch die im Inneren des Deckels der Schraubkappe 6' befindlichen Mitnehmerelemente als Antrieb des Schneidrings in Fig. 3C dargestellt.

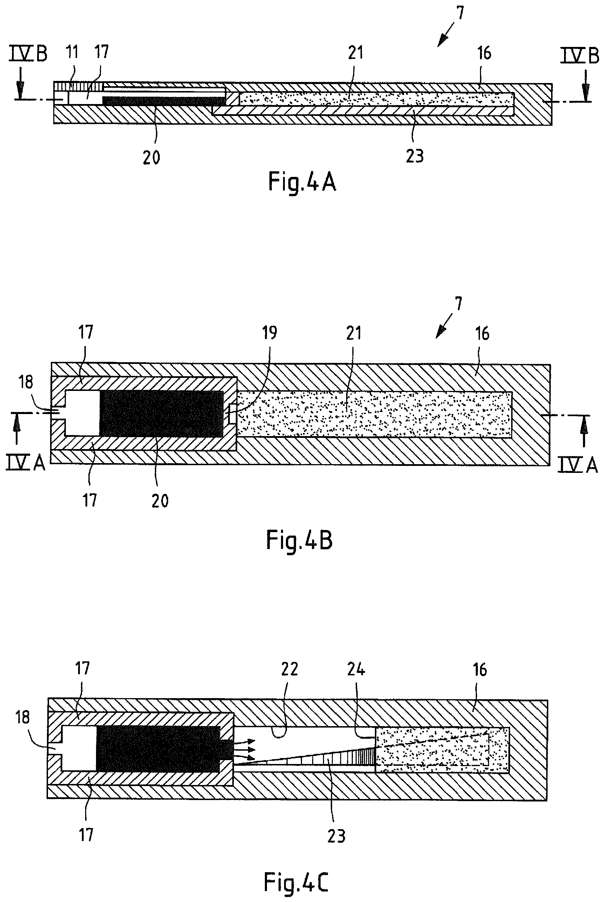

Fig. 4A zeigt ein Indikatorelement 7 im Vertikalschnitt entlang der Linie IV A - IV A aus Fig. 4B, welches beispielsweise in den Figuren 1 und 2 zum Einsatz kommt. Dieses Indikatorelement 7 verfügt über ein elektrochemisches Aktivierungselement 11, welches wie ein Feuchtigkeitssensor reagiert. Fig. 4A zeigt das Indikatorelement 7 im Horizontalschnitt entlang der Linie IV B - IV B aus Fig. 4A. Das Indikatorelement 7 weist zunächst ein Trägersubstrat 16 auf. Auf dem Trägersubstrat 16 sind elektrische Leiter 17 vorgesehen, die im Bereich eines stirnseitigen Endes voneinander beabstandet sind und dort einen Spalt 18 zueinander ausbilden. Dieser Spalt 18 kann im Bereich der Kavität 10A angeordnet sein und insbesondere bis in den Öffnungsbereich O hineinragen. Über dem Spalt 18 kann das Aktivierungselement 11 angeordnet sein und im Falle der Aktivierung den Spalt 18 kurzschließen.

Die beiden Leiter 17 sind auf der anderen Seite durch ein Auslöseelement 19 voneinander getrennt.

Wird ein elektrischer Kurzschluss über den Spalt 18 zwischen den beiden Leitern 17 hergestellt, z.B. über das Aktivierungselement 11, so kann das Auslöseelement 19 beispielsweise aufgelöst werden. Das Auslöseelement 19 kann beispielsweise eine galvanische Zelle sein, welche sich über den Kurzschluss zwischen den Leitern 17 entlang des Spalts 18 selbst auflöst.

Zu erkennen ist, dass das Auslöseelement 19 ein Elektrolyt 20 von einem Reaktionspartner 21 räumlich und mechanisch trennt. Beispielsweise ist der Elektrolyt NaCl. Der Reaktionspartner 21 kann beispielsweise eine nanoskalige Aluminiumschicht sein. Andere Substrate als Elektrolyten und Reaktionspartner sind ebenfalls denkbar.

Wird das Auslöseelement 19 geöffnet, so kann Elektrolyt 20 an den Reaktionspartner 21 gelangen. Es erfolgt eine Reaktion zwischen dem Elektrolyt 20 und dem Reaktionspartner 21. Diese Reaktion pflanzt sich insbesondere nach dem Prinzip einer lateralen Oxidation entlang des Kanals des Reaktionspartners 21 in einer Raumrichtung fort. Hierdurch wird der Reaktionspartner 21 aufgelöst. Das Auflösen des Reaktionspartners geschieht in Raumrichtung mit einer scharfen Front 24.

Fig. 4C zeigt, wie der Elektrolyt 20 in den Kanal 22 des Reaktionspartners 21 einströmt und wie dabei nach und nach der Reaktionspartner 21 mit dem Elektrolyt 20 reagiert. Durch diese Reaktion kann ein bedrucktes Label 23, was unterhalb des Reaktionspartners 21 angeordnet ist, erkennbar werden.

Beispielsweise kann das Label 23 mit Informationen zu einer Haltbarkeit bedruckt sein. Durch die fortlaufende Reaktion zwischen dem Elektrolyt 20 und dem Reaktionspartner 21 wird nach und nach weitere Information des Labels 23 freigelegt.

Die einmal angestoßene Reaktion ist nicht mehr aufzuhalten und der Reaktionspartner 21 wird vollständig aufgebraucht, so dass nach einer gewissen Zeit das gesamte Label 23 sichtbar ist.

Die Reaktionsgeschwindigkeit zwischen dem Elektrolyt 20 und dem Reaktionspartner 21 kann temperaturabhängig sein.

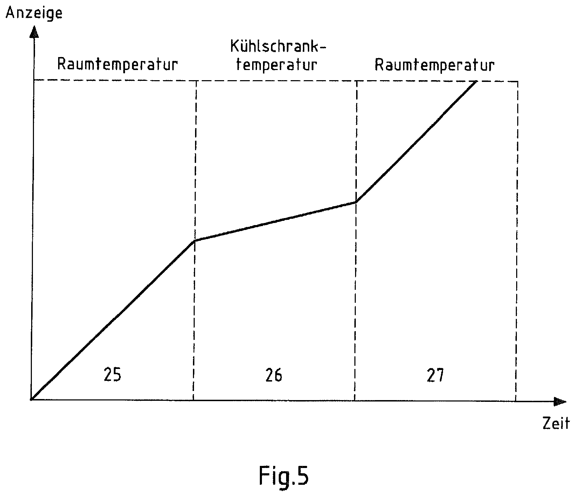

Fig. 5 zeigt beispielsweise die von der Front 24 der Reaktion zurückgelegte Strecke über die Zeit. In einem ersten Zeitfenster 25 ist die Packung beispielsweise bei Raumtemperatur aufbewahrt. Dies führt dazu, dass die Reaktion schneller abläuft und die Front 24 sich schneller fortpflanzt. In einem zweiten Zeitfenster 26 ist die Packung bei Kühlschranktemperatur gelagert. Zu erkennen ist, dass sich die Front 24 langsamer entwickelt. Im Zeitfenster 27 ist die Packung wieder bei Raumtemperatur gelagert und die Front 24 pflanzt sich wiederum schneller fort. Nach einer gewissen Zeit ist der gesamte Reaktionspartner 21 aufgebraucht und die Anzeige des Labels 23 ist vollständig abzulesen. Hierdurch kann beispielsweise angezeigt werden, dass das Produkt seine Mindesthaltbarkeit überschritten hat.

## Patentansprüche

1. Packung (1, 1'), insbesondere flüssigkeitsdichte Karton/Kunststoff-Verbundpackung für gießfähige Lebensmittelprodukte, mit einem wiederverschließbaren Öffnungselement (4, 4', 4", 4‴, 4ʺʺ), wobei das Öffnungselement (4, 4', 4", 4‴, 4ʺʺ) außen auf die Packung (1, 1') appliziert ist und/oder einen integralen Bestandteil der Packung (1, 1') bildet, sowie
(a) im Bereich des Öffnungselements (4, 4', 4", 4‴, 4ʺʺ) ein aktives Indikatorelement (7, 7', 7") vorgesehen ist, welches dem Verbraucher Informationen über den Zustand und/oder die Haltbarkeit des in der Packung (1, 1') befindlichen Produktes anzeigt,
(b) das Indikatorelement (7, 7', 7") ein Aktivierungselement (11, 11') aufweist, welches derart in oder am Öffnungselement (4, 4', 4", 4‴, 4ʺʺ) angeordnet ist, dass das Indikatorelement (7, 7', 7") beim erstmaligen Öffnen der Packung (1, 1') durch Betätigung des Öffnungselements (4, 4', 4", 4‴, 4ʺʺ) aktiviert wird, **dadurch gekennzeichnet, dass**
(c) das Aktivierungselement (11, 11') gleichzeitig als Messelement (12, 12') dient,
(d) das Aktivierungselement durch mechanische Bewegung zur Herbeiführung eines elektrischen Kontakts ausgelöst wird, der einen elektrochemischen Prozess in Gang setzt, und
(e) das Indikatorelement (7, 7', 7") wenigstens mittelbar durch einen oder mehrere der Parameter Zeit, Temperatur, pH-Wert, Sauerstoffgehalt und/oder durch Bio- oder Chemie-Rezeptoren beeinflussbar ist.

2. Packung nach Anspruch 1, **dadurch gekennzeichnet, dass** das Indikatorelement (7, 7', 7") und das Aktivierungselement (11, 11') einstückig ausgebildet sind.

3. Packung nach einem der Ansprüche 1 bis 2, **dadurch gekennzeichnet, dass** das Indikatorelement (7, 7', 7") wenigstens einen als Messelement (12, 12') dienenden Sensor aufweist.

4. Packung nach Anspruch 3, **dadurch gekennzeichnet, dass** das Indikatorelement (7, 7', 7") und das Messelement (12, 12') einstückig ausgebildet sind.

5. Packung nach einem der Ansprüche 1 bis 4, **dadurch gekennzeichnet, dass** das Indikatorelement (7, 7', 7") als flaches Etikett ausgebildet ist.

6. Packung nach Anspruch 5, **dadurch gekennzeichnet, dass** das Indikatorelement (7, 7') selbstklebend ausgebildet ist.

7. Packung nach einem der Ansprüche 1 bis 6, **dadurch gekennzeichnet, dass** das Indikatorelement (7, 7',7") durch zumindest einen Teil des Öffnungselements (4', 4", 4‴, 4‴) hindurch ablesbar ist.

8. Packung nach Anspruch 7, **dadurch gekennzeichnet, dass** das Öffnungselement (4, 4', 4", 4‴, 4ʺʺ) ein Sichtfenster (13) oder eine nicht-gefärbte Fläche zum Erkennen der Information des Indikatorelements (7, 7'; 7") aufweist.

9. Verfahren zur Herstellung einer Packung nach einem der Ansprüche 1 bis 8, umfassend die folgenden Schritte:
- Herstellen einer Verpackung aus einem Zuschnitt oder einer Materialbahn aus Verbundmaterial, Befüllen der Verpackung mit einem Produkt,
- Verschließen der gefüllten Verpackung zu einer Getränke- oder Lebensmittelpackung,
- Aufbringen des Indikatorelements auf den Giebelbereich der Packung und
- Aufbringen des Öffnungselements auf die Packung, wobei das Indikatorelement ganz oder teilweise vom Öffnungselement überdeckt ist.

10. Verfahren zur Herstellung einer Packung nach einem der Ansprüche 1 bis 8, umfassend die folgenden Schritte:
- Herstellen einer Verpackung aus einem Zuschnitt oder einer Materialbahn aus Verbundmaterial,
- Befüllen der Verpackung mit einem Produkt,
- Verschließen der gefüllten Verpackung zu einer Getränke- oder Lebensmittelpackung,
- Einbringen des Indikatorelements in das Öffnungselement und
- Aufbringen des Öffnungselements auf die Packung.

## Claims

1. Package (1), in particular liquid-tight cardboard/plastic composite package for pourable food products, having a resealable opening element (4, 4', 4", 4‴, 4ʺʺ), the opening element (4, 4', 4", 4‴, 4"") being applied to the outside of the package (1) and/or forming an integral part of the package (1), and also
(a) an active indicator element (7, 7', 7") is provided in the region of the opening element (4, 4', 4", 4""), which indicator element (7, 7', 7") displays information to the consumer about the condition and/or shelf life of the product contained in the package (1),
(b) the indicator element (7, 7', 7") has an activation element (11) which is arranged in or on the opening element (4, 4', 4", 4‴, 4"") in such a way that the indicator element (7, 7', 7") is activated when the package (1) is opened for the first time by actuation of the opening element (4, 4', 4", 4'", 4"")
**characterized in that**
(c) the activation element (11) simultaneously serves as a measuring element (12),
(d) the activating element is triggered by mechanical movement to bring about an electrical contact which initiates an electrochemical process, and
(e) the indicator element (7, 7', 7") is at least indirectly influenceable by one or more of the parameters time, temperature, pH, oxygen content and/or by bio- or chemical receptors.

2. Package according to claim 1, **characterized in that** the indicator element (7, 7', 7") and the activation element (11) are formed integrally.

3. Package according to any one of claims 1 to 2, **characterized in that** the indicator element (7, 7', 7") has at least one sensor serving as a measuring element (12).

4. Package according to claim 3, **characterized in that** the indicator element (7, 7', 7") and the measuring element (12) are formed in one piece.

5. Package according to any one of claims 1 to 4, **characterized in that** the indicator element (7, 7', 7") is formed as a flat label.

6. Package according to claim 5, **characterized in that** the indicator element (7, 7') is designed to be self-adhesive.

7. Package according to one of claims 1 to 6, **characterized in that** the indicator element (7, 7', 7") can be read through at least part of the opening element (4', 4", 4'", 4‴).

8. Package according to claim 7, **characterized in that** the opening element (4, 4', 4", 4‴, 4"") has a viewing window (13) or a non-colored surface for recognizing the information of the indicator element (7, 7'; 7").

9. Method of manufacturing a package according to any one of claims 1 to 8, comprising the following steps:
- Producing a package from a blank or web of composite material, filling the package with a product,
- Closing the filled package to form a beverage or food package,
- Applying the indicator element to the gable area of the package, and
- Applying the opening element to the package, wherein the indicator element is completely or partially covered by the opening element.

10. Method of manufacturing a package according to any one of claims 1 to 8, comprising the following steps:
- Making a package from a blank or web of composite material,
- Filling the package with a product,
- Closing the filled package to form a beverage or food package,
- Inserting the indicator element into the opening element, and
- Applying the opening element to the package.

## Revendications

1. Emballage (1), en particulier emballage composite carton/plastique étanche aux liquides pour des produits alimentaires pouvant être versés, avec un élément d'ouverture refermable (4, 4', 4", 4‴, 4ʺʺ), l'élément d'ouverture (4, 4', 4", 4‴, 4ʺʺ) étant appliqué à l'extérieur sur l'emballage (1) et/ou formant une partie intégrante de l'emballage (1), ainsi que
(a) dans la zone de l'élément d'ouverture (4, 4', 4", 4‴, 4"") est prévu un élément indicateur actif (7, 7', 7") qui indique au consommateur des informations sur l'état et/ou la durée de conservation du produit se trouvant dans l'emballage (1),
(b) l'élément indicateur (7, 7', 7") présente un élément d'activation (11) qui est disposé dans ou sur l'élément d'ouverture (4, 4', 4", 4‴, 4"") de telle sorte que l'élément indicateur (7, 7', 7") est activé lors de la première ouverture de l'emballage (1) par actionnement de l'élément d'ouverture (4, 4', 4", 4'", 4""),
**caractérisé en ce que**
(c) l'élément d'activation (11) sert en même temps d'élément de mesure (12),
(d) l'élément d'activation est déclenché par un mouvement mécanique pour provoquer un contact électrique qui déclenche un processus électrochimique, et
(e) l'élément indicateur (7, 7', 7") peut être influencé au moins indirectement par un ou plusieurs des paramètres temps, température, pH, teneur en oxygène et/ou par des récepteurs biologiques ou chimiques.

2. Emballage selon la revendication 1, **caractérisé en ce que** l'élément indicateur (7, 7', 7") et l'élément d'activation (11) sont formés d'une seule pièce.

3. Emballage selon l'une des revendications 1 à 2, **caractérisé en ce que** l'élément indicateur (7, 7', 7") comporte au moins un capteur servant d'élément de mesure (12).

4. Emballage selon la revendication 3, **caractérisé en ce que** l'élément indicateur (7, 7', 7") et l'élément de mesure (12) sont formés d'une seule pièce.

5. Emballage selon l'une des revendications 1 à 4, **caractérisé en ce que** l'élément indicateur (7, 7', 7") est réalisé sous forme d'étiquette plate.

6. Emballage selon la revendication 5, **caractérisé en ce que** l'élément indicateur (7, 7') est autocollant.

7. Emballage selon l'une des revendications 1 à 6, **caractérisé en ce que** l'élément indicateur (7, 7', 7") est lisible à travers au moins une partie de l'élément d'ouverture (4', 4", 4‴, 4'").

8. Emballage selon la revendication 7, **caractérisé en ce que** l'élément d'ouverture (4, 4', 4", 4'", 4"") comporte une fenêtre de visualisation (13) ou une surface non colorée pour la reconnaissance de l'information de l'élément indicateur (7, 7' ; 7").

9. Procédé de fabrication d'un emballage selon l'une quelconque des revendications 1 à 8, comprenant les étapes suivantes :
- Réalisation d'un emballage à partir d'une découpe ou d'une bande de matériau composite, Remplissage de l'emballage avec un produit,
- Fermeture de l'emballage rempli pour former un emballage de boisson ou de produit alimentaire,
- Application de l'élément indicateur sur la zone de pignon de l'emballage, et
- Application de l'élément d'ouverture sur l'emballage, l'élément indicateur étant entièrement ou partiellement recouvert par l'élément d'ouverture.

10. Procédé de fabrication d'un emballage selon l'une quelconque des revendications 1 à 8, comprenant les étapes suivantes :
- Réalisation d'un emballage à partir d'une découpe ou d'une bande de matériau composite,
- Remplissage de l'emballage avec un produit,
- Fermeture de l'emballage rempli pour former un emballage de boisson ou de produit alimentaire,
- Mise en place de l'élément indicateur dans l'élément d'ouverture et
- Application de l'élément d'ouverture sur l'emballage.
